# EUROPEAN PATENT APPLICATION

(11) **EP 4 653 072 A1**
(43) Date of publication of application: **26.11.2025**
(21) Application number: 24176917.3
(22) Date of filing: 21.05.2024
(51) Int. Cl.: B01D 29/33, B01D 35/027, B01D 35/28, C12M 1/26

(54) **FILTER ASSEMBLY FOR A FERMENTATION VESSEL AND A FERMENTATION VESSEL COMPRISING THE FILTER ASSEMBLY**

(71) Applicant: Gantenbrink, Bruno Alexander, 58708 Menden (DE); Popa, Vitalie, 6811 Bardar, Ialoveni (MD)
(72) Inventor: GANTENBRINK, Bruno A., 58708 Menden (DE); POPA, Vitalie, 6811 Bardar, Ialoveni (MD)
(74) Representative: DTS Patent- und Rechtsanwälte PartmbB

(57) **Abstract**

The present invention relates to a filter assembly for a fermentation vessel. Further, the present invention relates to a fermentation vessel comprising a filter assembly. In particular, the present invention relates to a fermentation vessel, comprising a filter assembly, for producing fermented products under constant, controlled pressure, preferably without adding additives and/or preservatives.

## Description

The present invention relates to a filter assembly for a fermentation vessel. Further, the present invention relates to a fermentation vessel comprising a filter assembly. In particular, the present invention relates to a fermentation vessel, comprising a filter assembly, for producing fermented products under constant, controlled pressure, preferably without adding additives and/or preservatives.

The traditional process of fermenting wine presents inherent challenges that have long plagued winemakers. One of the most significant hurdles is the risk of blockage within fermentation vessels, primarily attributed to the accumulation of byproduct gases produced during fermentation process. As the gas builds up, it exerts pressure on the vessel's interior, potentially leading to valve blockages and compromising the integrity of the fermentation process.

To deal with these problems, winemakers are using stainless steel fermentors. These are different from the old-style fermenting vessels or tanks (e.g. made of materials like wood or concrete) and come with lots of benefits that help overcome the problems of the old tanks. For example, stainless steel fermentors provide durability and corrosion resistance, which is crucial for maintaining quality over multiple fermentation cycles. Their smooth, non-porous surface allows for easy cleaning, reducing the risk of contamination and ensuring consistent quality of the wine. In addition, in stainless steel fermentors the pressure is controlled to prevent the valves from being blocked by the fermenting product such as CO₂. This is typically done by allowing the excess gas to escape gradually through a pressure release valve.

Stainless steel fermentors play an important role in the production of sparkling wines through their ability to manage secondary fermentation and facilitate controlled CO₂ release. During secondary fermentation, which is essential for imparting effervescence to sparkling wines, stainless steel fermentors provide a stable and hygienic environment for this process, allowing winemakers to monitor parameters like temperature and pressure carefully.

However, releasing carbon dioxide (CO₂) during fermentation is a crucial step in winemaking to prevent pressure buildup in the fermentor, which can lead to valve blockages and other issues. This process involves gradually releasing the excess CO₂ from the fermentor in controlled increments. Before releasing the pressure, winemakers must carefully determine the amount of CO₂ produced and the corresponding pressure levels inside the fermentor.

In addition, during winemaking processes solid particles (sediments) are settled at a bottom of the container (e.g. fermentation container or vessel) as a natural result of the fermentation process.

For example, in primary fermentation, sediments primarily comprise grape solids, such as skins, seeds, and pulp in winemaking, or solid particles from grains and hops in brewing. These sediments contribute to the initial fermentation process, including the extraction of flavors, colors, and tannins from the raw materials. In secondary fermentation, sediments typically comprise yeast cells, residual sugars, and other precipitates that settle out of the liquid as fermentation progresses. This phase often occurs after the initial fermentation and is common in processes like winemaking, where a secondary fermentation, such as malolactic fermentation, may take place.

To collect sediments, winemakers and brewers use various techniques depending on the specific process and scale of production. Common methods include racking, where the liquid is siphoned off from the sediment layer, or the use of filtration systems to separate the liquid from the solid particles. In some cases, sediment may be left undisturbed at the bottom of the vessel and the liquid carefully decanted off the top.

These methods for collecting sediments (e.g. racking, filtration, or decanting) can however lead to loss of product, oxygen exposure, and increased risk of contamination. These processes can also be time-consuming and may result in variations in product quality between batches.

It is therefore an object of the present invention to address the aforesaid problems and enhance the functionality of existing fermentation vessels (e.g. fermenters). Specifically, the present invention aims to improve the performance of fermenters by addressing challenges encountered during both primary and secondary fermentation processes, as well as operation at higher pressures. These challenges include the accumulation of sediments, which can lead to reduced efficiency, increased risk of contamination, and potential valve blockages for example due to the increased pressure in the fermentation vessels. By enhancing the functionality of fermenters, the invention aims to ensure smooth operation, minimize downtime, and maintain optimal conditions for fermentation, thereby improving the overall quality and yield of the final product.

The present invention provides for a filter assembly for a fermentation vessel, e.g. a tank or container for fermenting materials (e.g. grapes or vegetables for winemaking or grains for brewing). Specifically, the present invention relates to a fermentation vessel comprising a filter assembly.

In particular, a filter assembly is configured to prevent solid particles (sediments) within the fermentation container or vessel from entering into an area or chamber defined by the filter assembly within the container.

In particular, a container or (a fermentation vessel) is configured to be used in fermentation processes, such as brewing, winemaking, or the production of certain foods like yogurt or cheese.

In particular, a container is configured to continuously maintain a constant pressure during chemical processes involved in the production of alcoholic beverages. For example, in particular the chemical processes comprising maceration, primary fermentation, malolactic fermentation, aging and/or secondary fermentation.

In particular, a container (a fermentation vessel) may have various shapes and sizes depending on their intended purpose and the specific requirements of the fermentation process.

For example, common shapes include cylindrical, conical, and rectangular. For example, cylindrical vessels are often used for large-scale fermentation processes due to their efficient use of space and ease of cleaning. Conical vessels are popular in brewing and winemaking, as they facilitate the separation of sediment from the fermenting liquid. Rectangular tanks are used for specialized applications or when space constraints are a concern.

In particular, a container is configured to produce alcoholic beverages or fermentable materials under constant and controlled pressure, preferably without adding additives and/or preservatives.

In particular, a container can be made of various materials, such as stainless steel, glass, or even wood. For example, the container is a stainless-steel tank or a stainless steel fermentor.

In particular, the filter assembly is configured to be mounted in a container or a fermentation vessel.

In particular, the filter assembly is configured to be removably arranged in a container.

In particular, the filter assembly is configured to filter solids or solid substances from fermented products.

In particular, the filter assembly comprises a filter element or at least two filter elements configured to be assembled (connected) together. In this way, it is possible to adjust the dimensions of the filter assembly to the size of the container.

For example, the filter assembly comprises two filter elements stacked on top of each other or connected together (e.g. along a longitudinal axis) to enlarge or extend the length of the filter assembly along this longitudinal axis. This increases the overall length or height of the filter assembly in a vertical direction.

In particular, the filter assembly comprises a filter element or at least two filter elements having a tubular shape or a planner shape or a disc shape or a cone (tapered) shape.

In particular, the filter assembly, e.g. each of the filter elements, comprises openings for filtering the solid contents (e.g. for preventing solid contents to pass therethrough).

In particular, the filter assembly, e.g. a filter element or at least two filter elements, is configured to define a volume free of solid substances within the container.

In particular, the filter assembly is configured to protect a pressure release valve of a container (e.g. from solid substances).

In particular, the filter assembly configured to define a volume (or a channel) free of solid substances in a container, which volume is in communication with (fluidly connected to) a pressure release valve.

In particular, the filter assembly, e.g. a filter element, is configured to be positioned in a container at a close proximity to an inlet opening or an outlet opening of a container, wherein the filter element or the at least two filter elements preferably are of a planar shape with a dimeter fitted to the dimensions of the container (e.g. an inner diameter of the container)

Alternatively, the filter assembly, e.g. a filter element or at least two filter elements, is configured to be positioned centrally in a container, extending from an inlet opening of a container to an outlet opening of a container. Preferably, the filter element or at least two filter elements are of a tubular shape (e.g. forming a filter tube).

In particular, the filter element (or at least two filter elements stacked on top of each other) is positioned centrally in a container and is configured to define a lumen, a volume or a chamber free of solid contents within a container, e.g. at a centre of the container.

This volume or chamber extends from an inlet opening to an outlet opening of a container. In this way a separate, protected chamber for liquid fermented products is created within the container. Advantageously, solid particles or solid contents are prevented by the filter assembly from entering into the lumen or volume of the filter tube.

In particular, solid particles (sediments) that are settled at a bottom of the container (e.g. fermentation container or vessel) as a natural result of the fermentation process are prevented by the filter assembly from entering into the lumen or volume of the filter tube.

In particular, the filter assembly comprises a filter element (or at least two filter elements that are positioned in parallel) of planar shape. The filter element is configured to be positioned within a container along a transversal axis (e.g. a horizonal axis of a container). Preferably, the filter assembly (e.g. the filter element or at least two filter elements) is arranged at a position close to an inlet opening of the container and is configured to define a volume or a chamber free of solid contents within a container, e.g. at an upper portion or at a top position of the container.

In particular, the chamber is fluidly connected to a pressure release valve connected to a container, e.g. close to an opening of the container at a top position.

In particular, the filter assembly further comprises an inlet channel for introducing liquids or fermentation materials into a container. For example, grapes, grape juice, wort (unfermented beer), or any other liquid or material that needs to undergo fermentation.

In particular, the inlet channel is configured to be securely attached to a filter element, or at least two filter elements.

In particular, the inlet channel is configured to define a bypass channel (a distinct channel) within the container for introducing liquids or fermentation materials into the container.

In particular, the inlet channel comprises an inlet end which is configured to be sealed or closed by a closure element (a flange) and an outlet end which is disposed in an interior volume of the container.

In particular, the filter assembly is configured to define a first volume, e.g. via an inlet channel, for introducing raw materials into the container (solid and/or liquid) for the fermentation process, and a second volume, e.g. via a filter element and at least two filter elements, for fermented products in liquid form. In this way, the filter assembly is configured to filter out solid particles from entering into the second volume.

In particular, the second volume is devoid of solid contents and advantageously is in communication with a pressure control valve or a pressure release valve. In this way, the filter assembly is configured to protect the pressure control valves from blockage in excess of CO₂ or at higher pressures.

In particular, the container comprises an inlet opening for introducing the fermentation material.

In particular, the container comprises components for controlling temperature, pressure, agitation, and other factors that influence the fermentation process.

In particular, the container comprises valves for regulating the flow of materials.

In particular, the container comprises a pressure release valve for releasing excess pressure (e.g. due to CO₂ production) to prevent over-pressurization of the container.

In particular, the container comprises tubes for collecting or draining liquids during the fermentation process.

In particular, the container comprises at least one pressure release valve and at least one port for filling and discharging fluids from the container.

In particular, the container may further comprise additional filters that are installed in the outlet opening to remove any solids or impurities from the fermented product before it is collected.

In particular, the container may further comprise taps (or spigots) that are configured to be attached to the outlet opening and allow for easy collection of the fermented product without needing to open the container.

In particular, the container comprises a temperature control system, which includes heating elements, cooling jackets, or external temperature controllers configured to regulate the temperature of the fermentation process.

In particular, the container further comprises an agitator or stirrer, which is designed to keep the fermentation mixture well-mixed and homogenous throughout the process, promoting uniform fermentation and preventing stratification of ingredients.

In particular, the container may further comprise pH and oxygen sensors, which provide valuable data for controlling and optimizing the fermentation process by monitoring pH levels and oxygen levels during fermentation.

In particular, the container comprises a sampling port for (periodic) sampling of the fermenting mixture without the need to open the container. This feature is useful for monitoring progress and adjusting the fermentation process.

In particular, the container comprises an aeration system for introducing oxygen or other food-graded inert gases into the fermentation mixture in the container during the fermentation process.

In particular, the container comprises a membrane element configured be in contact with the fermented product within the container.

In particular, the container comprises a stainless-steel tank and the membrane is a wooden membrane. Advantageously, the wood allows small amounts of oxygen to interact with the fermented product, which can influence its flavor and aging process.

In particular, the container comprises an outlet opening for removing the fermented product.

In winemaking, a layer of grape skins, seeds, and other solids (the "cap") is formed, for example, during the process of making red wine. The cap floats to the top of the fermentation vessel.

This cap however needs to be managed to ensure proper extraction of color, flavor, and tannins from the grape skins.

Traditionally, this was done mechanically by "punching down" or "pumping over" the cap to submerge it into the fermenting wine. Newer techniques involve the use of carbon dioxide (CO₂) produced during fermentation. The CO2 is blown through the cap. This method helps keep the cap moist and aids in the extraction process while also minimizing the risk of oxidation.

The disadvantages of mechanical cap management methods, such as punching down or pumping over, include the potential for oxygen exposure, inconsistent extraction of flavors, labor intensiveness, and a risk of contamination. These methods require manual labor and can introduce oxygen into the fermenting wine, leading to oxidation and spoilage. Additionally, they may result in uneven extraction of color and flavor compounds from the grape skins, affecting the quality of the final wine. Using patents to blow CO2 through the cap helps mitigate some of these issues but may still require additional equipment and energy consumption.

By way of the present filter assembly, the blockage of the ports, valves or a pressure release valve can be advantageously prevented.

For example, the cap can be managed through the filter assembly in the form of a filter tube (e.g. shaped as a ring), a filter cone or a filter disc. In particular, the fermented products within the filtered zine, e.g. the wine column within the filter tube, allows easy escape of excess CO2.

It is shown:
- **Fig. 1**: an exemplary schematic diagram of a filter assembly.
- **Fig. 2**: an exemplary schematic diagram of a filter assembly mounted in a container.
- **Fig. 3**: another exemplary schematic diagram of a filter assembly mounted in a container.
- **Fig. 4**: yet another exemplary schematic diagram of a filter assembly mounted in a container.
- **Fig. 5**: yet another exemplary schematic diagram of a filter assembly mounted in a container.
- **Fig. 6**: a picture of a filter assembly.
- **Fig. 7**: another picture of a filter assembly, indicating a filter element and an inlet channel in an open state.
- **Fig. 8**: yet another picture of a filter assembly, indicating a filter element and an inlet channel closed in a closed state;
- **Fig. 9**: several options for a filter;
- **Fig. 10**: an embodiment with several cylindrical filters in a row;
- **Fig. 11**: an embodiment with a cone formed filter; and
- **Fig. 12**: an embodiment with a flat formed filter.

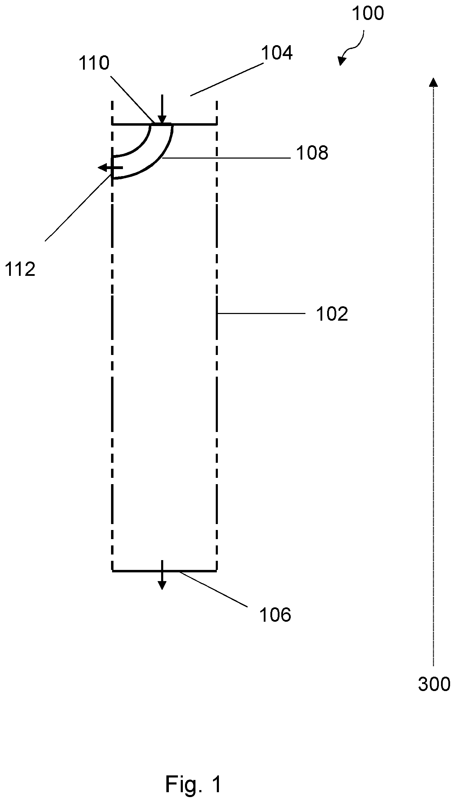

Fig. 1 shows an exemplary schematic diagram of a filter assembly.

The filter assembly comprises a filter element and an inlet channel attached to the filter element.

The filter element comprises a plurality of openings for preventing solid particles to pass therethrough.

The inlet channel for example comprises, for example, a tube which is fixed to the filter element by any suitable process, such welding or threaded connections.

The tube can be straight or curved.

The inlet channel is configured to define a separate channel or passage way (a bypass channel) for introducing raw materials for the fermentation process in the container (shown with arrows in Fig. 1).

The inlet channel comprises an inlet end and an outlet end (an opposite end).

The inlet end is configured to be closed by any suitable closure element (not shown in Fig. 1).

The filter element is configured to define a volume or chamber (filtered zone or volume) free of solid particles in the container, e.g. the in the filter zone solid particles cannot be entered.

The chamber for example is configured to be in fluid communication with a pressure release valves or inlet/outlet ports disposed in the container.

The chamber defined by the filter element comprises an inlet and/or an outlet allowing the passage of liquids therethrough.

In this example, the filter element is of a cylindric shape or a tube shape.

The filter tube comprises an upper end, a lower end and a body portions extending between the upper and lower ends.

The chamber or filtered zone is defined by the cylindrical filter body. The chamber comprise an outlet positioned, for example, at the lower end of the filter assembly.

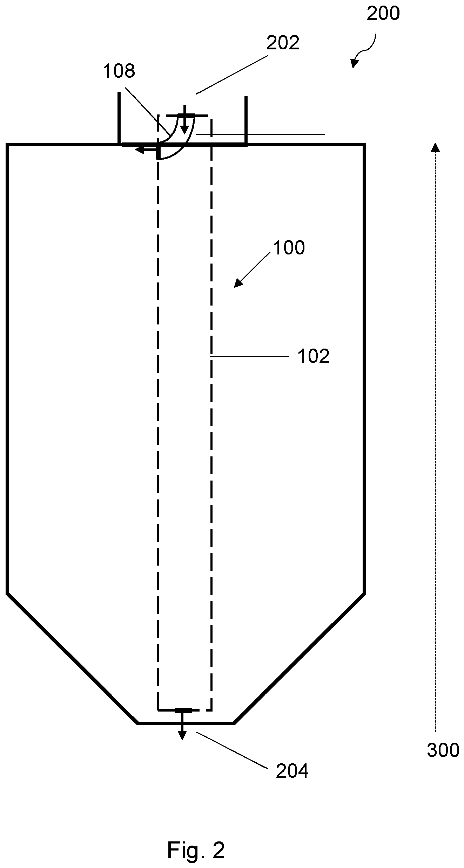

Fig. 2 shows an exemplary schematic diagram of a filter assembly mounted in a container (or a fermentation vessel).

The container comprises a fermentation vessel, where the fermentation process takes place.

The container comprises (or is equipped with) components for controlling and monitoring the fermentation processes which are not shown. For example, the container comprises temperature and pH sensors for monitoring conditions, an agitator or stirrer to ensure uniform mixing of the fermentation broth. Additionally, the container comprises a plurality of ports for adding raw materials or sampling the fermenting mixture.

The container may comprise a cylindrical, conical, rectangular container. The choice of shape of the container in particular depends on factors such as the type of fermentation, the volume of product to be produced, and the available space in the production facility.

The container comprises a pressure control regulator and/or a pressure release valve for controlling and monitoring the pressure in the container during the fermentation process

The container is configured to maintain a constant and controlled atmosphere, e.g. free from contaminations and undesired oxygen, during the fermentation processes.

The filter assembly is similar to the one shown in Fig. 1. The filter assembly comprises a filter element having a tubular shape which is centrally positioned in the container.

The filter element at the upper end is in communication with an inlet opening of the container, and at the lower end it is in fluid communication with an outlet opening of container.

The inlet channel is disposed at the upper end of the filter element (filter tube).

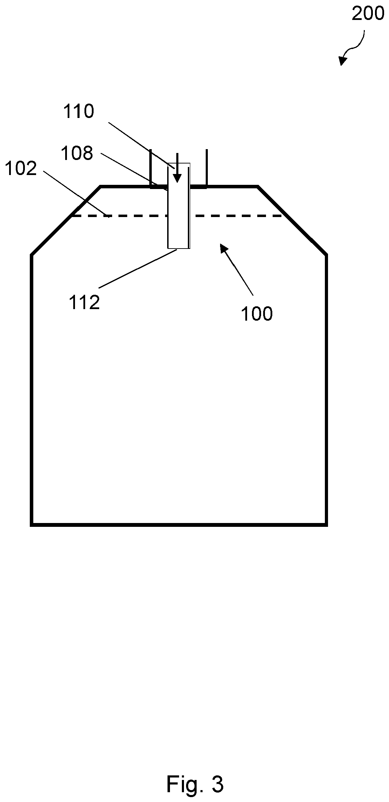

Fig. 3 shows another exemplary schematic diagram of a filter assembly mounted in a container.

In this example, the filter assembly comprises a planar filter element and an inlet channel connected thereto.

The filter assembly is disposed at upper portion of the container, i.e. close to the inlet opening of the container.

The filter zone is positioned in this example upper stream from the planar filter element.

The container functions as a fermentation vessel and is configured to perform fermentation processes at controlled and constant temperature and/or pressure. To this end, the container comprises, for example, components explained above with respect to Fig. 2 that are not shown.

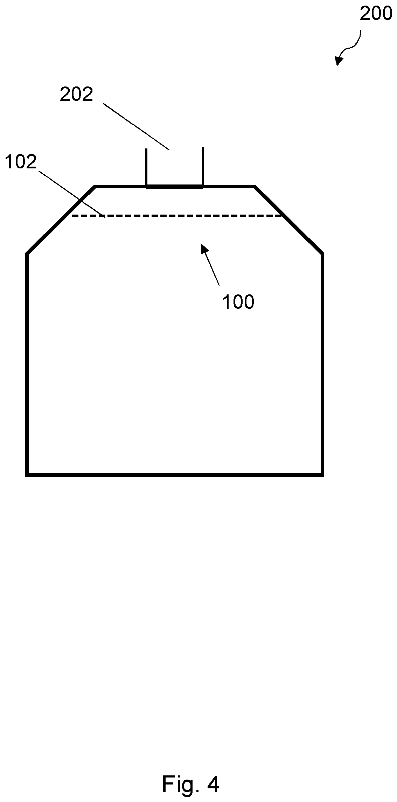

Fig. 4 shows another exemplary schematic diagram of a filter assembly mounted in a container (or a fermentation vessel).

The filter assembly in this example comprise a filter element or at least two filter elements positioned in parallel.

The filter element or the filter elements are of a planar or circular or disc shape.

The filter element is arranged in the container in a direction perpendicular to a longitudinal axis of the container.

The filter element is configured to prevent the solid particles from entering into the filtered zone located at upper portion of the container.

The filtered zone (volume) is in fluid communication with an inlet opening or a pressure release valve (not shown) of the container.

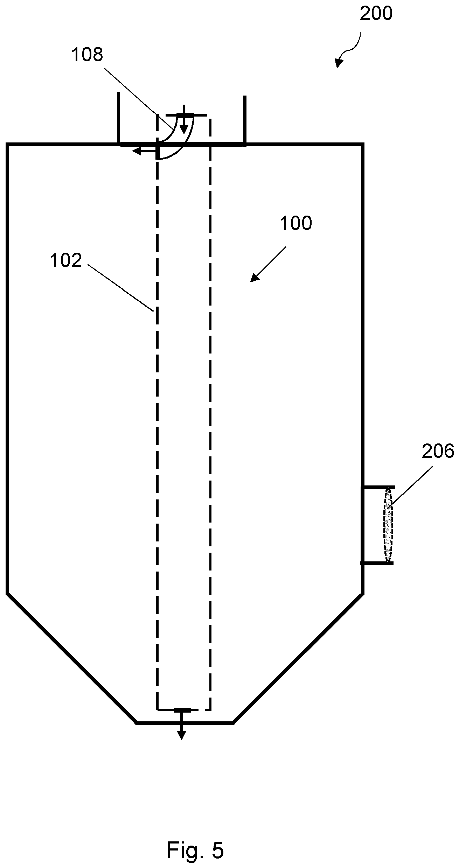

Fig. 5 shows yet another exemplary schematic diagram of a filter assembly mounted in a container.

In this example, the filter assembly is of a tubular shape. However, the filter assembly of other shapes as shown in the preceding figures can be also used.

The container further comprises a membrane, preferably a wooden membrane. The wooden membrane is configured to contact liquid or the fermented products in the container.

In this example, the membrane is positioned for example at lower portion of the container.

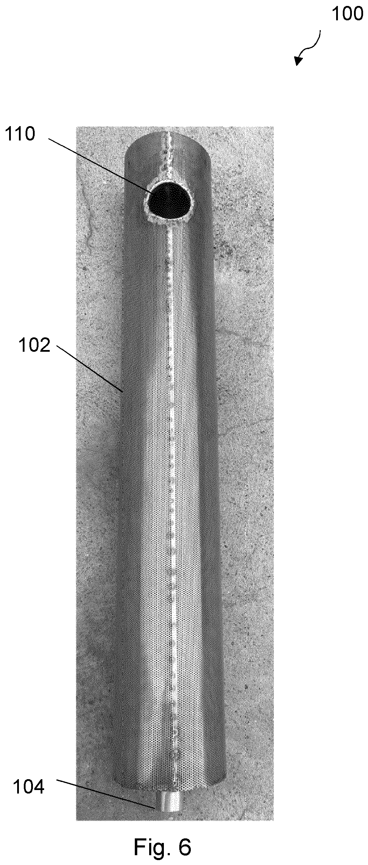

Fig. 6 shows a picture of a tubular filter assembly.

The filter element comprises a cylindrical body (including openings) and an outlet at a lower portion.

An outlet end of the inlet channel is visible in Fig. 6.

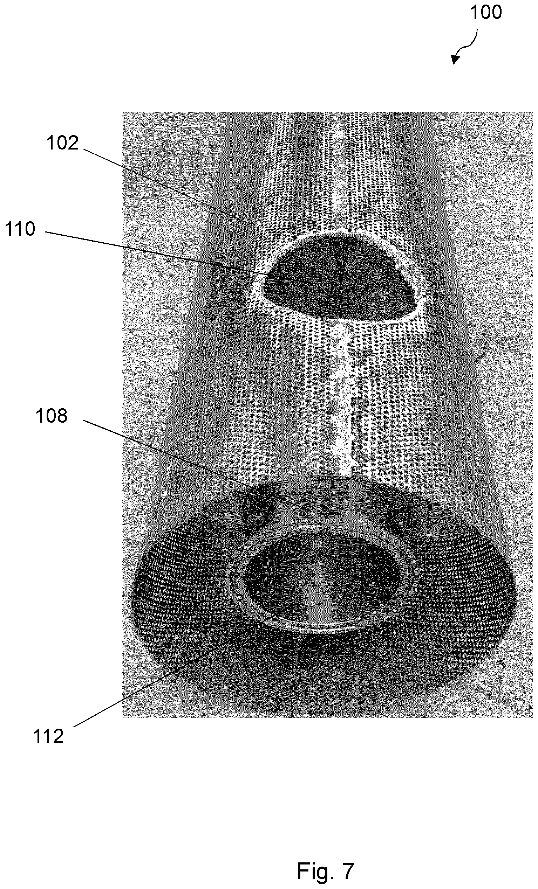

Fig. 7 shows another picture of a filter assembly, partly indicating a filter element and an inlet channel in an open state.

The inlet channel is fixed to the filter element (e.g. the cylindrical body of the filter tube) by means of three pins or securing elements.

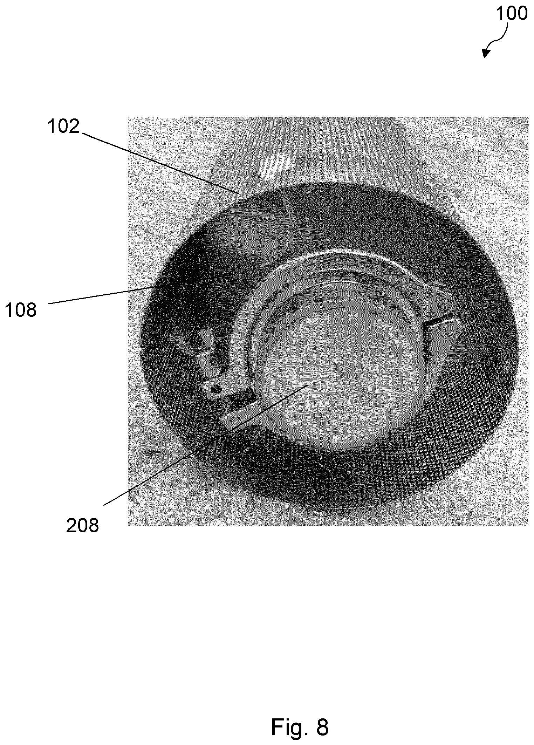

Fig. 8 shows yet another picture of a filter assembly, partly indicating a filter element and an inlet channel closed in a closed state.

In Fig. 8 the outlet end of the inlet channel is closed by a flange element.

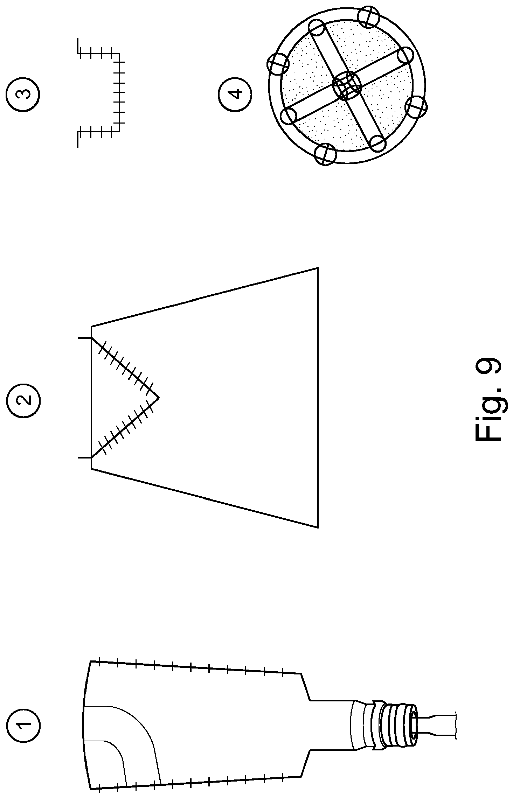

Fig. 9 shows several possible geometries of a filter (element).

The advantages of the filter can be described as follows:
1. The filter is unique and universal (and doesn't exist in the market) to be used for stainless steel tanks, pressure stainless tanks, and wooden vessels using auric infinity technology (cf. https://auric-infinity.com/ and e.g. https://magazine.wein.plus/winemaking-without-cellar-sulphur-and-additives-revolution-in-the-wine-barrel) (but also for any fermentable vessel made out of any suitable material like stainless steel, concrete, glass, plastic... etc,).
2. The filter is for any size of a vessel.
3. The filter can represent one piece of the equipment or consist of many parts assembled.
4. The filter can have different geometrical shapes as well as sizes to perfectly fit the vessel size and shape and also be installed in such a place inside the vessel that will protect the Safety relief valve and Pressure relief valve inlet from blocking which will be placed inside the upper part of the vessel.
5. The filter can also be used to drain the liquid out of the vessel without any oxidation, contamination or evaporation using liquid gravity or gas pressure.
6. The filter can be permanently mounted inside the vessel or be removable.
7. The filter can be a perforated filter or wire mesh filter (https://blog.wstyler.com/filters/perforated-plate-vswoven-wire-mesh-filtersis ).
8. The filter prevents the skins, pulp, seeds, and stems of the fruit (grape marc, apple marc....etc) from blocking the pressure relief valve and/or safety relief valve during the full process:
   1. Maceration - Fermentation - Malolactic Fermentation - Aging (because the wooden barrels will not be able to hold big pressure values (for example 5 Bar), especially big wooden barrels).
   2. Maceration - Fermentation - Malolactic Fermentation - Aging - Second fermentation (in case of the filter being used for pressure stainless steel they can withstand high pressure).

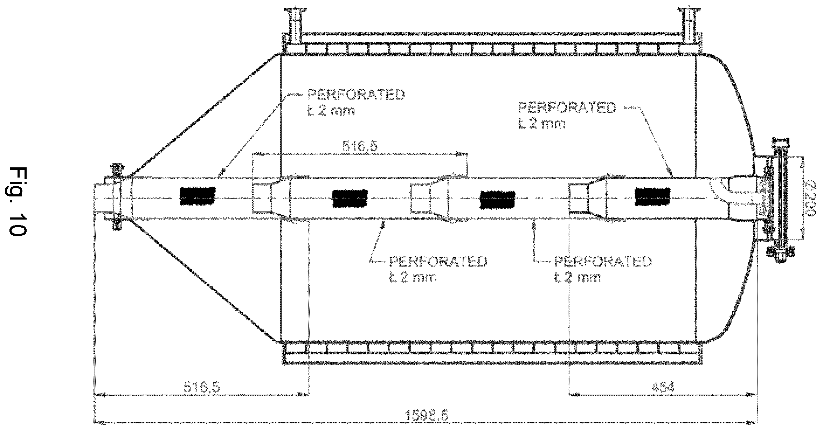

Fig. 10 shows an embodiment with several cylindrical filters in a row arranged in a stainless steel tank.

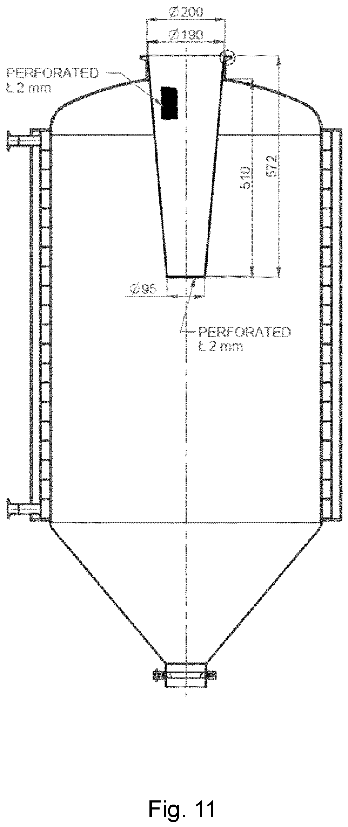

Fig. 11 shows an embodiment with a cone formed filter.

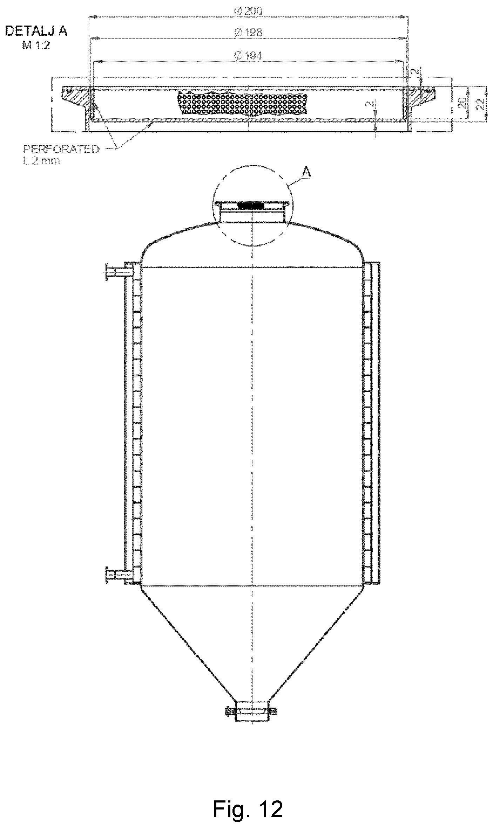

Fig. 12 shows an embodiment with a flat formed filter.

### Reference numerals

- 100: filter assembly
- 102: filter element
- 104: inlet
- 106: outlet
- 108: inlet channel
- 110: inlet end
- 112: outlet end
- 200: container
- 202: inlet opening
- 204: outlet opening
- 206: membrane element
- 208: closure element
- 300: longitudinal axis

## Claims

1. A filter assembly (100) for a fermentation vessel (200) for producing fermentable materials under constant and controlled pressure,
the filter assembly (100) being configured to be mounted in the fermentation vessel (200),
wherein the filter assembly (110) comprises at least one filter element (102) configured to create a filtered zone free of solid contents in the fermentation vessel (200), preferably the filtered zone is configured to be in communication with an inlet (104) of the fermentation vessel (200) throughout chemical processes occurring in the fermentation vessel (200).

2. The filter assembly (100) according to claim 1,
**characterized in that**
the at least filter element (102) comprises openings configured to prevent the solid contents from entering to the filtered zone.

3. The filter assembly (100) according to claim 1 or 2,
**characterized in that**
the at least one filter element (102) comprises a cylindrical shape, a conical shape, a rectangular shape or a disc shape,
preferably the at least one filter element (102) is configured to be positioned close to an inlet (104) the fermentation vessel (200).

4. The filter assembly (100) according to any one of the preceding claims, **characterized in that**
the at least one filter element (102) comprising two or more filter elements that are configured to be connected along a longitudinal axis (300).

5. The filter assembly (100) according to any one of preceding claims, **characterized in that**
the filter assembly further comprises an inlet channel (108) configured to define a passage for raw materials, wherein the passage is district from the filtered zone.

6. The filter assembly (100) according to claim 5,
**characterized in that**
the inlet channel (108) comprises an inlet end (110) and an outlet end (112), wherein the inlet end (110) is configured to be sealed by a closure element (208) and the outlet end (112) is configured to be disposed outside the filtered zone.

7. The filter assembly (100) according to one claim 5 or 6,
**characterized in that**
the inlet channel (108) is configured to be integrally formed in, or securely attached to, the at least one filter element (102).

8. The filter assembly (100) according to any one of the preceding claims, **characterized in that**
the at least one filter element (102) comprises a cylindrical shape that is configured to be extended from an inlet (104) to an outlet (106) of the fermentation vessel (200).

9. The filter assembly (100) according to claim 8,
**characterized in that**
the at least one flirter element (102) comprises an upper end portion, a lower end portion and a cylindrical body portion extending between the upper and lower end portions,
wherein the cylindrical body portion comprises openings, and
wherein the cylindrical body portion is configured to create the filtered zone.

10. The filter assembly (100) according to claim 8 or 9,
**characterized in that**
the upper end portion of the at least one filter element (102) is configured to be in fluid communication with the inlet (104) of the fermentation vessel (200), and the lower end portion of the at least one filter element (102) is configured to be in fluid communication with the outlet (106) of the fermentation vessel (200).

11. A fermentation vessel (200) comprising a filter assembly (100) according to any one of the preceding claims,
wherein the fermentation vessel is configured to continuously maintain a constant pressure and controlled atmosphere, preferably free from undesired oxygen, during chemical processes occurring in the fermentation vessel (200), wherein the filter assembly (100) is configured to create a fluid communication with an inlet (104) of the fermentation vessel (200) throughout the chemical processes, and
wherein the chemical processes comprise maceration, fermentation, malolactic-fermentation, and aging processes, preferably the chemical processes further comprise a second fermentation process.

12. The fermentation vessel (200) comprising a filter assembly (100) to claim 11, **characterized in that**
the fermentation vessel (200) further comprises at least one pressure release valve, and/or ports for filling and discharging fluids during the chemical processes,
wherein the filter assembly (100) is configured to protect the at least one pressure release valve and/or the ports from blockage by solid contents.

13. The fermentation vessel (200) according to claim 11 or 12,
**characterized in that**
the at least one filter element (102) comprises a body that is configured to be partially arranged in the inlet (104) of the fermentation vessel (200).

14. The fermentation vessel (200) according to any one of claims 11 to 13, **characterized in that**
the inlet end (110) of the channel (108) is configured to be arranged at the inlet (104), and the outlet end (112) of the channel (108) is configured to be arranged outside the filtered zone, thereby creating a bypass passage for introducing raw materials into the fermentation vessel (200).

15. The fermentation vessel (200) according to any one of the preceding claims, **characterized in that**
the fermentation vessel (200) further comprises a membrane element (206), preferably a wooden membrane, wherein the membrane element (206) is configured to contact liquid or fermented materials in the fermentation vessel (200), preferably the fermentation vessel (200) is made of stainless steel.
